# EUROPÄISCHE PATENTSCHRIFT

(11) **EP 2 220 214 B1**
(45) Veröffentlichungstag und Bekanntmachung des Hinweises auf die Patenterteilung: **17.01.2018**
(21) Anmeldenummer: 08860440.0
(22) Anmeldetag: 10.12.2008
(51) Int. Cl.: A61K 35/14

(54) **VERFAHREN ZUR ERHÖHUNG DER IMMUNREAKTIVITÄT**
METHOD FOR INCREASING IMMUNOREACTIVITY
PROCÉDÉ D'AUGMENTATION DE LA RÉACTIVITÉ IMMUNITAIRE

(30) Priorität: 10.12.2007 AT 19962007
(43) Veröffentlichungstag der Anmeldung: 25.08.2010
(60) Teilanmeldung: 17203941.4
(73) Patentinhaber: Medizinische Universität Innsbruck, 6020 Innsbruck (AT); Apeiron Biologics AG, 1030 Wien (AT)
(72) Erfinder: BAIER, Gottfried, A-6020 Innsbruck (AT); LOIBNER, Hans, A-1230 Vienna (AT); SCHUSTER, Manfred, A-2191 Schrick (AT); LAMETSCHWANDTNER, Günther, A-1030 Wien (AT); WOLF, Dominik, A-6162 Mutters/Raitis (AT)
(74) Vertreter: Sonn & Partner Patentanwälte
(86) Internationale Anmeldenummer: PCT/AT2008/000443
(87) Internationale Veröffentlichungsnummer: WO 2009/073905

(56) Entgegenhaltungen:
- WO-A-2008/033403
- WO-A2-2005/069791
- US-A1- 2007 015 768
- CHIANG JEFFREY Y ET AL: "Ablation of Cbl-b provides protection against transplanted and spontaneous tumors." THE JOURNAL OF CLINICAL INVESTIGATION APR 2007, Bd. 117, Nr. 4, April 2007 (2007-04), Seiten 1029-1036, XP002527157 ISSN: 0021-9738 in der Anmeldung erwähnt
- LAMETSCHWANDTNER GUENTHER ET AL: "Development of an Effective Cancer Immune Therapy by Cbl-b Silencing" JOURNAL OF IMMUNOTHERAPY, Bd. 31, Nr. 9, November 2008 (2008-11), Seite 943, XP008106012 & 23RD ANNUAL MEETING OF THE INTERNATIONAL-SOCIETY-FOR-BIOLOGICAL-THERA PY-OF-CANCER; SAN DIEGO, CA, USA; 20081030, ISSN: 1524-9557
- PLAUTZ G E ET AL: "T-cell adoptive immunotherapy of metastatic renal cell carcinoma.", UROLOGY OCT 1999, vol. 54, no. 4, October 1999 (1999-10), pages 617-623 ; dis, ISSN: 1527-9995
- OBERMANN SONJA ET AL: "Peptide-beta2-microglobulin-major histocompatibility complex expressing cells are potent antigen-presenting cells that can generate specific T cells.", IMMUNOLOGY SEP 2007, vol. 122, no. 1, September 2007 (2007-09), pages 90-97, ISSN: 0019-2805
- HARTUNG H P ET AL: "The role of interferon-gamma in the pathogenesis of experimental autoimmune disease of the peripheral nervous system.", ANNALS OF NEUROLOGY MAR 1990, vol. 27, no. 3, March 1990 (1990-03), pages 247-257, ISSN: 0364-5134
- EGUCHI AKIKO ET AL: "Efficient siRNA delivery into primary cells by a peptide transduction domain-dsRNA binding domain fusion protein.", NATURE BIOTECHNOLOGY JUN 2009, vol. 27, no. 6, June 2009 (2009-06), pages 567-571, ISSN: 1546-1696
- REINHARD HINTERLEITNER ET AL: 'Adoptive Transfer of siRNA Cblb-Silenced CD8+ T Lymphocytes Augments Tumor Vaccine Efficacy in a B16 Melanoma Model' PLOS ONE Bd. 7, Nr. 9, 04 September 2012, XP055141808 DOI: 10.1371/journal.pone.0044295 ISSN: 1932-6203
- STROMNES INGUNN M ET AL: "Abrogating Cbl-b in effector CD8(+) T cells improves the efficacy of adoptive therapy of leukemia in mice.", THE JOURNAL OF CLINICAL INVESTIGATION OCT 2010, vol. 120, no. 10, October 2010 (2010-10), pages 3722-3734, ISSN: 1558-8238
- Marc Oliver Salzberg,: "Adoptive cellular immunotherapy with APN401, autologous Cbl-b-silenced peripheral blood mononuclear cells, in patients with solid tumors.", ASCO University , 9 February 2017 (2017-02-09), Retrieved from the Internet: URL:http://meetinglibrary.asco.org/content /170952-176 [retrieved on 2017-02-09]

## Beschreibung

Die vorliegende Erfindung betrifft Verfahren zur Modulation der Immunantwort von Zellen.

Aktive Immunisierung ermöglichte erst die flächendeckende Bekämpfung bedrohlichster Infektionskrankheiten und erreichte sogar in einigen Fällen deren weltweite Ausrottung unter Verwendung eines kostengünstigen und hoch effizienten körpereigenen Abwehrmechanismus. Es wurden und werden daher Anstrengungen unternommen um gegen diverse Indikationen prophylaktische und therapeutische Vakzinierungsansätze zu entwickeln. Allerdings bedingt eine effiziente Impfung die Induktion einer Immunantwort, welche zu einer schützenden Immunität führt. Mangelnde Immunogenität des Immunisierungsantigens führt jedoch zum Ausbleiben des gewünschten Effektes. Hochinteressante und spezifische Antigenformulierungen wurden bereits zur Prophylaxe sowie zur Behandlung von Malaria, HIV, Influenza oder Tumorerkrankungen, um nur einige prominente Beispiele anzuführen, entwickelt. Allerdings blieb der Erfolg dieser Therapien, beispielsweise bedingt durch mangelnde Immunogenität des Immunisierungsantigens aus. Ferner stehen selbst breit angewendete Impfstoffe vor Problemen mangelnder Immunogenität, wie zum Beispiel die Hepatitis B Vakzine, die nur für etwa 80 % der Behandelten auch tatsächlich schützende Immunantwortstiter aufbauen. Der wesentliche Grund für das Ausbleiben der Reaktivität des Immunsystems ist, dass diese Antigene nicht als "fremd" erkannt werden. Im Säugetier sind es vor allem T-Zellen welche entscheiden, ob eine durch Antigen präsentierende Zellen (APCs) dargebotene Struktur als körpereigen oder als fremd erkannt wird. Um eine Immunantwort zu induzieren, sind im Wesentlichen zwei getrennte und voneinander unabhängige Signale nötig. Dieser Mechanismus soll ein Überschießen des Immunsystems verhindern. Erste Voraussetzung ist, dass der T-Zell-Rezeptor das durch die APC dargebotene Antigen auch erkennt. Ist dies nicht der Fall, findet keine weitere Reaktion statt. Ferner ist zur Induktion einer Immunantwort die Interaktion des CD28 Rezeptors auf der T-Zelloberfläche mit B7, welches auf der APC nur dann exprimiert wird, wenn diese die antigene Struktur als gefährlich einstuft, zwingend erforderlich Im Falle einer Vakzinierung mit einem nur marginal immunogenen Impfantigen kann die Kostimulation über die Interaktion zwischen B7 und CD28 ausbleiben, was in weiterer Folge zu keiner Immunantwort, sondern vielmehr zum Auftreten von Toleranz auf T-Zellebene führt. Es konnte allerdings gezeigt werden, dass die Notwendigkeit der Kostimulation durch Abschalten eines Enzyms, der E3-Ubiquitinligase Cbl-b umgangen werden kann. Dieses Enzym stellt einen entscheidenden Weichenstellpunkt in der Steuerung der Immunreaktivität dar (Chiang et al., J Clin Invest (2007) 117(4): 1033-1034, doi:10.1172/JCI29472). In Abwesenheit von Cbl-b können verabreichte aber kaum immunogene Substanzen zur Induktion einer starken Immunantwort führen. Ferner sind Cbl-b defiziente Mäuse (homozygotischer Gen knock-out) lebensfähig und deren Immunsystem ist in der Lage effizient autolog-induzierte Tumore zu erkennen und dagegen eine vor allem auf CD8+ T-Zellenberuhende lytische Immunantwort aufzubauen (Loeser et al., JEM (2007) doi:10.1084/iem.20061699). Allerdings führte die beschriebene, gänzliche Abschaltung des Enzyms ebenfalls zu einer erhöhten Autoimmunität, nach Immunisierung mit Superantigenen. Loeser at al. somit konnten aufzeigen, dass Cbl-b als negativer Regulator maßgeblich für die "Immunreaktivität" von T-Zellen verantwortlich ist.

siRNA Technologie zur Attenuierung der spezifischen Gen-Expression ist auch für Cbl-b mit geringerer Effizienz bereits beschrieben. Die US 2007/0087988 betrifft ein Verfahren zur Regulierung der HPK1, dessen Expression durch die Erhöhung der Cbl-b Expression erhöht werden kann und vice versa (z.B. durch Cbl-b siRNA Inhibition).

Die US 2007/0054355 beschreibt Cbl-b Peptide und Cbl-b assoziierte Proteine, insbesondere POSH, und deren Verwendung zur Behandlung von Cbl-b assoziierten Krankheiten.

WO 2004/078130 A2 betrifft Zusammensetzungen zur Behandlung von POSH assoziierten Krankheiten, wie Viruserkrankungen, Krebs oder neurologische Störungen. POSH kann u.a. zusammen mit einer Vielzahl von POSH-assoziierten Proteinen, darunter auch Cbl-b, zur Verfügung gestellt werden.

Die US 2006/0292119 A1 betrifft Verfahren zur Erhöhung der Immunantwort von Immunzellen durch Inhibierung negativer Immunregulatoren in der Zelle. Solche negativen Immunregulatoren sind ausgewählt aus Proteinen welche mit der molekularen Stabilität in Verbindung stehen, z.B. durch Ubiquitinierung, Deubiquitinierung und Sumoylierung, sowie aus Transkriptionsfaktoren, welche die Expression von NFkB-Inhibitoren induziert, oder Suppressoren der Transkription von NkB-Zielgenen.

Die US 2007/0015768 A1 offenbart Verbindungen zur Inhibierung von Ubiquitin-Ligasen, mitunter eine siRNA zur Unterdrückung der Cbl-b Expression.

Plautz G et al. (Urol. 54 (4) (1999): 617-623) beschreibt ein ex vivo Verfahren zur Aktivierung von Patienten-isolierten CD4 und CD8 T-Lymphozyten, wobei die Immunzellen mit IL-2/staphylococcal enterotoxin A (SEA) behandelt wurden und danach wieder in den Patienten injiziert wurden.

Es wurde allerdings noch keine für klinische Anwendungen geeignete Anwendung von Cbl-b Mediatoren beschrieben. Es ist daher ein Ziel der vorliegenden Erfindung ein praxistaugliches Verfahren zur Modulierung der Immunreaktivität zur Verfügung zu stellen.

Die vorliegende Erfindung betrifft daher ein in vitro oder ex vivo Verfahren zur Erhöhung der Immunreaktivität von Zellen des Immunsystems, umfassend die transiente Reduzierung oder Inhibierung der Expression von Cbl-b durch Verwendung einer kurzen DNA und/oder RNA Sequenz komplementär zu einem Teil der Ziel-Cbl-b-mRNA Sequenz, wobei die Zellen vor oder nach der Entnahme mit einem Antigen kontaktiert wurden, und wobei die Immunreaktivität der Zellen gegen das Antigen erhöht wird, und wobei die Zellen antigenpräsentierende Zellen, PBMCs (Peripheral Blood Mononuclear Cells), T-Lymphozyten, B-Lymphozyten, Monozyten, Makrophagen, NK Zellen und/oder NKT Zellen umfassen.

Das Cbl-b Gen sowie dessen Genprodukte sind im Stand der Technik ausführlich beschrieben (UniGene Id. Hs.3144 und Hs.381921). Cbl-b Sequenzen sind z.B. in der GenBank Datenbank unter den Acc. Nr. NM_008279 und NP_009112 veröffentlicht. Anti-Cbl-b Antikörper, siRNAs und Antisense-Inhibitoren sind kommerziell erhältlich. Bestimmte siRNAs geeignet zur Reduzierung oder Inhibierung der Cbl-b Expression und damit auch der Cbl-b Funktion sind beispielsweise in der US 2007/0054355 mit gemischten RNA/DNA Nukleotiden und einer Länge von ca. 20 Basen offenbart.

Um der Gefahr einer Überreaktion des Immunsystems, welche zur Induktion von z.B. Autoimmunreaktivität führen kann, zu begegnen, darf die Hemmung/Abschaltung von Cbl-b Funktionen in T-Zellen nur in einem zeitlich streng definierten Zeitraum erfolgen. Es ist daher für einen therapeutisch adjuvanten Vakzinierungsansatz essentiell, Cbl-b nur für einen begrenzten Zeitraum kontrolliert zu attenuieren, um den Aufbau einer Immunisierungsantigen spezifischen Immunantwort zu unterstützen jedoch durch rechtzeitige Wiederherstellung des immunologischen "Normalzustandes" einer Autoimmunerkrankung vorzubeugen. Daher wird erfindungsgemäß nur eine bestimmte Auswahl von isolierten Zellen des Immunsystems in vitro oder ex vivo behandelt und in einen Patienten wieder zurückgeführt. Ein Ansatz zur effizienten in vitro oder ex-vivo Cbl-b Attenuierung ist daher die Voraussetzung für die Steigerung der Immunreaktivität.

Erfindungsgemäß wird die Cbl-b Funktion durch Reduzierung oder Inhibierung der Expression von Cbl-b reduziert oder inhibiert. Die Begriffe Reduzierung oder Inhibierung betreffen die Absenkung der Funktion (bzw. der Expression) von Cbl-b im Vergleich zur unveränderter, natürlicher Funktion bis zur kompletten Inhibierung der Funktion. Vorzugsweise wird die Funktion (bzw. Expression) um mindestens 30%, 40%, 50%, 60%, 70%, 80%, 90% oder 95% reduziert.

Die Reduzierung oder Inhibierung der Funktion von Cbl-b erfolgt transient. D.h. die Funktion wird nur vorläufig w.o. angegeben reduziert und kann sich in Folge dessen wieder erholen, z.B. durch Verbrauch oder Abbau von Inhibitoren, wie z.B. Cbl-b siRNA, oder durch Neubildung oder nicht Cbl-b beeinträchtigter Zellen (in vivo). Die transiente Reduzierung von Cbl-b in Immunzellen kann dabei auch repetitiv erfolgen, z.B. bis ein therapeutischer Erfolg erzielt wurde.

Vorzugsweise wird die Expression von Cbl-b durch Verwendung von Cbl-b antisense RNA oder siRNA reduziert oder inhibiert. Hierzu werden kurze DNA und/oder RNA Sequenzen komplementär zu einem der Teil der Ziel-Cbl-b-mRNA Sequenz eingesetzt, so dass diese damit hybridisieren und inaktivieren. Die Länge dieser Sequenzen ist vorzugsweise mindestens 15, 18, 20, 22, 25, 28, 30, 35, 40, 50, 60, 70, 80, 90, 100, 120, 140, 160, 180 oder 200 Basen bis zur kompletten Ziel Sequenz, vorzugsweise bis zu 2502, 2000, 1500, 1000, 500 oder 300 Basen. Vorzugsweise werden die Sequenzen der SEQ ID Nr. 1, 2, 3, 4, 5, 6, 7 und/oder 8 verwendet.

Offenbarungsgemäß kann die Funktion von Cbl-b durch eine Vielzahl weiterer bekannter Komponenten reduziert oder inhibiert werden, wie beispielsweise durch Verwendung von Cbl-b Antagonisten, Inhibitoren, insbesondere Aptameren oder Intrameren. Jegliche Antagonisten oder Inhibitoren, die die Wirkung bzw. die Funktion von Cbl-b unterdrücken können offenbarungsgemäß verwendet werden um die Immunreaktivität der Zellen erhöht werden. In einer weiteren Ausführungsform der vorliegenden Offenbarung werden vorzugsweise Antagonisten oder Inhibitoren verwendet zur Herstellung eines pharmazeutischen Mittels zur in vitro, ex vivo aber auch in vivo Erhöhung der Immunreaktivität von Zellen des Immunsystems. Ermöglicht werden Behandlungen von Krankheiten mit supprimierten oder ineffizientem Immunsystem, insbesondere Krebs, sowie die Erhöhung der Immunantwort gegenüber (Impf)antigenen, die mit den Zellen des Immunsystems in vivo oder ex vivo kontaktiert werden können.

Die vorliegende Offenbarung betrifft ebenfalls ein Verfahren zur Reduzierung der Immunreaktivität von Zellen des Immunsystems umfassend die Reduzierung oder Inhibierung der c-Cbl Funktion der Zellen, wodurch die Immunreaktivität der Zellen gegen das Antigen reduziert wird, vorzugsweise durch transiente Reduzierung oder Inhibierung, insbesondere durch Verwendung von c-Cbl antisense RNA oder siRNA. Zur Steigerung der Immunreaktivität ist es nicht zwingend nötig, c-Cbl gemeinsam mit Cbl-b zu attenuieren. Wie in den Beispielen gezeigt erbringt die Attenuierung von c-Cbl vielmehr eine Umkehr der durch Cbl-b Inhibierung herbeigeführten Effekte. Cbl-b und c-Cbl haben daher antagonistische Funktionen. C-Cbl erfüllte ferner eine bisher noch unbekannte Funktion in der Feinregulierung der T-Zell Reaktivität, indem dessen Attenuierung zu einer erhöhten Immuntoleranz führt. Daher eignet sich die Reduzierung oder Inhibierung der c-Cbl Funktion zur Immunsuppression und ermöglicht daher dessen Anwendung bei z.B. Inflammation oder Allergien. Da das Ausmaß und die Richtung der Attenuierung von der Dosis der Reduzierung oder Inhibition von Cbl-b bzw. c-Cbl (analog uzu Cbl-b wie hierin beschrieben) abhängt, können beide Faktoren in ihrer Funktion in Kombination reduziert werden. Zur Erhöhung der Immunreaktivität überwiegt die Reduktion von Cbl-b über der Reduktion von c-Cbl und umgekehrt zur Erniedrigung. C-Cbl antisense oder siRNA können die gleichen Sequenzlinien wie oben für Cbl-b beschrieben aufweisen. Vorzugsweise werden die Sequenzen der SEQ ID Nr. 9, 10, 11, 12, 13, 14, 15 und/oder 16 verwendet.

Speziell, werden in besonderen Ausführungsformen der Offenbarung Zellen verwendet, die das Antigen aufgenommen haben und vorzugsweise ein Antigenfragment präsentieren oder ein Antigenfragment im Kontext von HLA besser erkennen und dadurch aktiviert werden.

In bevorzugten Ausführungsformen sind die erfindungsgemäß einzusetzenden Zellen antigenpräsentierende Zellen, PBMCs (Peripheral Blood Mononuclear Cells), T-Lymphozyten, B-Lymphozyten, Monozyten, Makrophagen und/oder dendritische Zellen, insbesondere T-Lymphozyten, CD8+ T-Lymphozyten, CD4+ T-Lymphozyten, insbesondere Th1, Th2, Th17, Tregs (regulatory T cells) oder CTL (cytotoxische T-Zellen), NK Zellen oder NKT Zellen. Ebenso ist die Verwendung von CD3/CD19-negativen Lymphozyten im allgemeinen möglich. NK Zellen bilden ein besonders bevorzugte Gruppe hiervon. Vorzugsweise wurde das Antigen durch die Zellen aufgenommen und diese präsentieren vorzugsweise ein Antigenfragment. Insbesondere bevorzugt werden dabei PBMCs und T-Zellen in Kombination zum dadurch Behandeln, um eine besonders starke Antigen-spezifische Reaktion hervorzurufen. In anderen Ausführungsformen, insbesondere zur allgemeinen Erhöhung der Immunreaktivität (z.B. zur Behandlung einer Immuninsuffizienz) reichen diverse T-Zellen alleine aus, um eine breite Wirkung zu erzielen. Die erfindungsgemäß erhöhte Immunreaktivität wird vorzugsweise durch diese Zellen, insbesondere CD8 oder CD4, sowie NK bzw. NKT Zellen, vermittelt.

Zur Transfektion der Zellen, insbesondere der T-Zellen, oder der NK Zellen, mit einem Cbl-b Inhibitoren, wie Cbl-b siRNA oder einem knock-out Cbl-b Konstrukt, wird vorzugsweise die Elektroporation eingesetzt. Hierzu können beliebige Medien eingesetzt werden, die zu einer Transfektion führen, also zur Inhibition von Cbl-b geeignet sind. Ein solches Medium ist beispielsweise Optimem (Gibco, #31985-047).

Zusätzlich können die Zellen noch mit einem immunstimulierenden Substanzen, z.B. einem immunstimulierenden Cytokin oder Liganden weiterer immunstimmulierender Rezeptoren (wie TLRs, toll like receptor) oder Antikörper gegen Oberflächenmoleküle, vorzugsweise CD3 und/oder CD28, behandelt werden bzw. stimuliert werden um eine Immunreaktion durch die Zellen zu fördern.

Ebenfalls kann die Inhibition von cbl-b im Rahmen einer durch dendritische Zellen gestützte Vakzinierung, vorzugsweise einer anti-Tumor Vakzinierung, eingesetzt werden.

Alternativ bzw. zusätzlich ist auch die in vitro Kokultivierung von cbl-b inhibierten Zellen mit vom Patienten gewonnenen dendritischen Zellen, die vorzugsweise mit Tumor(zell)antigenen beladen wurden, möglich, sowie die Verwendung der Ko-Kultur für die erfindungsgemäßen Zwecke.

"Vakzinierung" wie hierin verwendet soll nicht im absoluten Sinn - also einer Verabreichung eines Immunogens, welches zu einem absoluten Schutz durch das Immunsystem führt - verstanden werden, sondern als immunologische Verabreichung um den Schutz durch das Immunsystem zu erhöhen bzw. das Immunsystem, insbesondere die Zellen desselben gegen das Vakzinantigen zu aktivieren.

In einem besonderen Aspekt betrifft die vorliegende Erfindung eine pharmazeutische Zusammensetzung umfassend eine kurze DNA und/oder RNA Sequenz komplementär zu einem Teil der Ziel-Cbl-b-mRNA Sequenz zur Anwendung in einer Therapie umfassend Isolierung von Zellen des Immunsystems eines Patienten, wobei die Zellen antigenpräsentierende Zellen, PBMCs (Peripheral Blood Mononuclear Cells), T-Lymphozyten, B-Lymphozyten, Monozyten, Makrophagen, NK Zellen und/oder NKT Zellen umfassen; in vitro oder ex vivo Erhöhung der Immunreaktivität der Zellen durch Verwendung der kurzen DNA und/oder RNA Sequenz komplementär zu einem Teil der Ziel-Cbl-b-mRNA Sequenz, und Reimplantation der Zellen in den Patienten, wobei durch eine transiente Reduktion oder die Inhibition der Cbl-b Funktion der Zellen die Immunreaktivität erhöht wird. Weiters betrifft die Erfindung Isolierte Zellen des Immunsystems eines Patienten, die mit einer kurzen DNA und/oder RNA Sequenz komplementär zu einem Teil der Ziel-Cbl-b-mRNA Sequenz transient transfiziert wurden, wobei die Zellen antigenpräsentierende Zellen, PBMCs (Peripheral Blood Mononuclear Cells), T-Lymphozyten, B-Lymphozyten, Monozyten, Makrophagen, NK Zellen und/oder NKT Zellen umfassen; und wobei die Zellen vor oder nach der Entnahme mit einem Antigen kontaktiert wurden, zur Anwendung in einer Therapie umfassend die Reimplantation der Zellen in den Patienten, wobei durch eine transiente Reduktion oder die Inhibition der Cbl-b Funktion der Zellen die Immunreaktivität erhöht wird.

Eine Realisierung der, vorzugsweise zeitlich limitierten, Steigerung der Immunreaktivität begleitend zu einer Vakzinierung, der Verabreichung des Antigens, kann durch eine Herabsetzung der Cbl-b Expression in einem geringen Anteil der zirkulierenden T-Zellen herbeigeführt werden. Hierfür können PBMCs (periphere mononukleare Blutzellen) aus Vollblut bzw. Blutzellen aus dem Knochenmark und aus dem Tumorgewebe selbst (TILs) des Patienten, welcher im Idealfall einige Tage zuvor, z.B. 5 Tage immunisiert wurde, gewonnen und in vitro oder ex vivo mit einem Cbl-b spezifischen siRNA Attenuierungsansatz behandelt werden. Dieses Verfahren erfolgt sehr schnell. Im Idealfall könnte diese Zellpräparation nur wenige Minuten nach deren Entnahme dem Patienten wieder verabreicht werden. Optional können die Zellen durch für die jeweiligen Zellen geeignete ex vivo-Stimulations-Protokolle vermehrt oder expandiert werden, bevor die Zellen reimplantiert werden. Die in vitro aktivierten T-Zellen, welche nur wenige Prozent der T-Zellpopulation des Patienten darstellen, treffen bei deren Zirkulation im Empfängerorganismus in Lymphknoten auf Antigen präsentierende Zellen, welche bedingt durch die bereits erfolgte Immunisierung Antigene aufgenommen haben und dorthin migriert sind. Nachdem die in vitro behandelten T-Zellen kein Ko-Stimulationssignal benötigen, werden diese sofort nach Erkennung des Immunisierungsantigens proliferieren und Zytokine ausschütten, welche systemisch zur Induktion einer Immunantwort sowohl auf zellulärer als auch auf humoraler Ebene beitragen. Mit diesem Ansatz werden selbst schwach immunogene Antigene zur Etablierung eines lang anhaltenden Impfschutzes führen. Ebenso kann somit die Abstoßung autologer Tumoren in Krebspatienten eingeleitet werden. Ein Cbl-b-Antagonist steigert hierbei die Immunreaktivität, wobei diesen entweder bei einer alleinigen, bzw. bei einer begleitenden Chemo/Radiotherapie Behandlung von onkologischen Erkrankungen, oder in Kombination mit Passiv-Immunisierungen, wie tumorantigenspezifischen Antikörpern zur Anwendung kommt.

Dieses Verfahren ist daher zur Anwendung bei der Behandlung einer angeborenen oder erworbenen Immuninsuffizienz, insbesondere AIDS, multiples Myelom, chronische lymphatische Leukämie, medikamentenbedingte Immunsuppression oder einer Krebserkrankung, gegebenenfalls unter Auswahl von Krankheits-spezifischen Antigenen, geeignet. Insbesondere bevorzugt ist dabei die Behandlung einer Krebserkrankung, die feste Tumore bildet.

Zur Erhöhung der Erfolgschancen einer Therapie erfolgt die Behandlung einer Krebserkrankung vorzugsweise in Kombination mit einer weiteren Anti-Tumor-Therapie, insbesondere einer Chemotherapie, Radiotherapie, Verabreichung eines therapeutischen Biologics oder Dentritische-Zellen gestützter (Tumor)vakzinierung. Die Inhibition von cbl-b kann im Rahmen einer durch dendritische Zellen gestützte Vakzinierung, vorzugsweise einer anti-Tumor Vakzinierung, eingesetzt werden. Alternativ bzw. zusätzlich ist auch die in vitro Kokultivierung von cbl-b inhibierten Zellen mit vom Patienten gewonnenen dendritischen Zellen, die vorzugsweise mit Tumor(zell)antigenen beladen wurden, möglich, sowie die Verwendung der Ko-Kultur für die erfindungsgemäßen Zwecke.

Die in vitro oder ex vivo Erhöhung der Immunreaktivität kann im therapeutischen Verfahren wie oben beschrieben vorgenommen werden, wobei die Exposition der Zellen gegenüber einem Antigen bereits vor Entnahme der Zellen erfolgen kann oder nachher.

Die zeitliche Abfolge der Darbietung des Immunisierungsantigene, deren Aufnahme durch Antigen präsentierende Zellen und ferner der Migration dieser Zellen zu lokalen Lymphknoten, wo die aufgenommenen Substanzen reaktiv gemachten T-Zellen präsentiert werden, ist bei der therapeutischen Umsetzung zusätzlich bedeutend. Daher wird vorzugsweise der Patient mit dem Antigen beimpft, vorzugsweise noch vor der Isolierung der Zellen, insbesondere bevorzugt vor mindestens 1, 2, 3, 4 oder 5 Tagen und/oder höchstens 20, 16, 12, 10, 9, 8, 7, 6, 5, 4, 3, 2 oder 1 Woche(n) vor Isolierung der Zellen. Offenbart ist auch eine nachträgliche Vakzinierung oder eine Behandlung der Zellen in vitro oder ex vivo mit einem Antigen möglich.

Zusätzlich ist eine Verwendung von Antigen-präsentierenden Zellen möglich, die vorzugsweise vom Patienten selbst stammen und mit entsprechendem Antigen kontaktiert werden können und dann gemeinsam oder kurz vor oder nach der Gabe von cbl-b inhibierten Immunzellen, vorzugsweise T-Zellen, zur Erhöhung der spezifischen Immunreaktivität beitragen.

Vorzugsweise sind die Zellen spezifisch für ein bestimmtes Antigen oder es werden Zellen umfassend ein bestimmtes Antigen auf die Spezifität oder Anwesenheit des Antigens selektioniert, wobei die Immunreaktivität der selektionierten Zellen erhöht wird. Durch die Auswahl eines bestimmten Antigens, bzw. der Zellen mit immunsteigernder Spezifität hierfür, kann gezielt eine Immunreaktion gegen ein bestimmtes Ziel im Patienten gerichtet werden. Ein solches Ziel ist insbesondere ein Tumor (durch die Auswahl zumindest eines oder mehrerer Tumor-Antigene) oder ein Pathogen.

Es ist vorstellbar, dass parallel zur Abtrennung der Zellen im selben, sterilen Einwegröhrchen bereits die dementsprechende siRNA im Transfektionsansatz vorgelegt wird. Daher betrifft die vorliegende Offenbarung einen vorzugsweise sterilen Behälter, wie ein Einwegröhrchen, umfassend einen Cbl-b Inhibitor, insbesondere zur Erhöhung der Immunreaktivität von Zellen des Immunsystems gegen ein Antigen.

Ebenso sieht die vorliegende Offenbarung einen Kit vor, der einen Behälter, insbesondere ein Einwegröhrchen zum Aufnehmen der Zellen des Immunsystems, sowie einen Cbl-b-Inhibitor, wie siRNA, umfasst, insbesondere zur Erhöhung der Immunreaktivität von Zellen des Immunsystems gegen ein Antigen nach dem erfindungsgemäßen Verfahren.

Der Behälter bzw. der Kit kann auch zusätzlich ein immunstimulierende Substanzen, vorzugsweise Cytokin(e) oder Liganden anderer Rezeptoren (z.B. TLRs) oder Antikörper gegen Oberflächenmoleküle, vorzugsweise CD3 und/oder CD28, umfassen, um die Stimmulierung noch zusätzlich zu verstärken. Ebenso können der Kit oder der Behälter nach Stabilisierungskomponenten, -medien oder -puffer (zur Stabilisierung der Zellen), Transfektions-oder -Nukleofektions-Lösungen, vorzugsweise Zellmedien, wie RPMI oder Optimem, umfassen.

Die vorliegende Erfindung wird durch die folgenden Figuren und Beispiele illustriert ohne darauf beschränkt zu sein.

In den Figuren zeigen:
Fig. 1 eine schematische, vereinfachte Darstellung der T-Zellaktivierung durch Ko-Stimulation (a), oder im Falle der Attenuierung der Cbl-b-Expression durch alleinige Stimulation des T-Zell-Rezeptors (c), die üblicherweise nicht zur Aktivierung führt (b);
Fig. 2 eine Western-Blot-Analytik der Cbl-b-Expression. CD8⁺-Zellen wurden aus humanen PBMC isoliert, mit Cbl-b-siRNA transfiziert, in Kultur gehalten und mit einer Kontrollgruppe ohne, nach anti-CD3 oder nach anti-CD3 und anti-CD28-Stimulation verglichen. Als Ladekontrolle wurde Fyn verwendet;
Fig. 3 die IFN-gamma Sekretion humaner CD8⁺ Zellen 2 Tage nach siRNA Behandlung. Gemessen wurde die IFN-gamma-Konzentration in Überständen von CD8⁺-T-Zellen ohne Stimulation (Medium) (links) und nach CD3-spezifischer (Mitte) oder nach CD3- und CD28-spezifischer Ko-Stimulation (rechts). Es wurden jeweils 2 Tage alte Zellpopulationen verglichen, die mittels unspezifischer (1. Balken), Cbl-b spezifischer (2. Balken), c-Cbl-spezifischer (3. Balken) und Cbl-b- und c-Cbl-spezifischer siRNA (4. Balken) transfiziert wurden;
Fig. 4 die IL2 Sekretion humaner CD8⁺ Zellen 20 Tage nach siRNA Behandlung. Gemessen wurde die IL2-Konzentration in Überständen von CD8⁺-T-Zellen ohne Stimulation (Medium) (links) und nach CD3-spezifischer (Mitte) oder nach CD3- und CD28-spezifischer Ko-Stimulation (rechts). Es wurden jeweils 2 Tage alte Zellpopulationen verglichen, die mittels unspezifischer (1. Balken), Cbl-b spezifischer (2. Balken), c-Cbl-spezifischer (3. Balken) und Cbl-b- und c-Cbl-spezifischer siRNA (4. Balken) transfiziert wurden; und
Fig. 5 die zeitliche Abfolge des in vitro Cbl-b-Attenuierungsansatzes zur Steigerung der Immunreaktivität.
Fig. 6 die siRNA Aufnahme humaner T-Zellen, isoliert aus PBMCs (A), die siRNA Aufnahme CD8-Zell abgereicherter PBMCs (B)
Fig. 7 die Cbl-b mRNA Expression nach RNAi (A) und produzierte Cbl-b Proteinmenge nach RNAi in einem Western Blot (B)
Fig. 8 die IFN-gamma, TNF-alpha, IL-2 Produktion nach Cbl-b Inhibierung
Fig. 9 die IFN-gamma Produktion nach Cbl-b Inhibierung als Zeitprofil
Fig. 10 die Steigerung der T-Zell-Reaktivität gemessen durch CD107a+CD69 (A), CD107a, CD3, CD40L, ICAM (B) Markerexpression.
Fig. 11 A: Tumorwachstum in Mäusen nach Therapie, mit/ohne Cbl-b Unterdrückung in therapeutischen CD8 Zellen; B: Mortalität von Mäusen mit EG7ova Tumoren nach Therapie.

### Beispiele:

### Beispiel 1: Sequenzen

Die folgenden siRNA-Sequenzen wurden zur Inhibierung von Cbl-b verwendet, alleine oder in Kombination:
1. Sense Sequenz:
   G.A.A.C.A.U.C.A.C.A.G.G.A.C.U.A.U.G.A.U.U (SEQ ID Nr. 1) Antisense Sequenz:
   5'-P.U.C.A.U.A.G.U.C.C.U.G.U.G.A.U.G.U.U.C.U.U (SEQ ID Nr.2)
2. Sense Sequenz:
   G.U.A.C.U.G.G.U.C.C.G.U.U.A.G.C.A.A.A.U.U (SEQ ID Nr. 3) Antisense Sequenz:
   5'-P.U.U.U.G.C.U.A.A.C.G.G.A.C.C.A.G.U.A.C.U.U (SEQ ID Nr. 4)
3. Sense Sequenz:
   G.G.U.C.G.A.A.U.U.U.U.G.G.G.U.A.U.U.A.U.U (SEQ ID Nr. 5) Antisense Sequenz:
   5'-P.U.A.A.U.A.C.C.C.A.A.A.A.U.U.C.G.A.C.C.U.U (SEQ ID Nr. 6)
4. Sense Sequenz
   U.A.U.C.A.G.C.A.U.U.U.A.C.G.A.C.U.U.A.U.U (SEQ ID Nr. 7) Antisense Sequenz
   5'-P.U.A.A.G.U.C.G.U.A.A.A.U.G.C.U.G.A.U.A.U.U (SEQ ID Nr. 8)

Die folgenden siRNA-Sequenzen wurden zur Inhibierung von C-Cbl verwendet, alleine oder in Kombination:
1. Sense Sequenz
   A.A.U.C.A.A.C.U.C.U.G.A.A.C.G.G.A.A.A.U.U (SEQ ID Nr. 9) Antisense Sequenz
   5'-P.U.U.U.C.C.G.U.U.C.A.G.A.G.U.U.G.A.U.U.U.U (SEQ ID Nr. 10)
2. Sense Sequenz
   G.A.C.A.A.U.C.C.C.U.C.A.C.A.A.U.A.A.A.U.U (SEQ ID Nr. 11) Antisense Sequenz
   5'-P.U.U.U.A.U.U.G.U.G.A.G.G.G.A.U.U.G.U.C.U.U (SEQ ID Nr. 12)
3. Sense Sequenz
   U.A.G.C.C.C.A.C.C.U.U.A.U.A.U.C.U.U.A.U.U (SEQ ID Nr. 13) Antisense Sequenz
   5'-P.U.A.A.G.A.U.A.U.A.A.G.G.U.G.G.G.C.U.A.U.U (SEQ ID Nr. 14)
4. Sense Sequenz
   G.G.A.G.A.C.A.C.A.U.U.U.C.G.G.A.U.U.A.U.U (SEQ ID Nr. 15) Antisense Sequenz
   5'-P.U.A.A.U.C.C.G.A.A.A.U.G.U.G.U.C.U.C.C.U.U (SEQ ID Nr. 16)

### Beispiel 2: Transiente Reduktion der Cbl-b Expression

In diesem Beispiel wird gezeigt, dass die Immunreaktivität von T-Zellen ex vivo beeinflusst werden konnte.

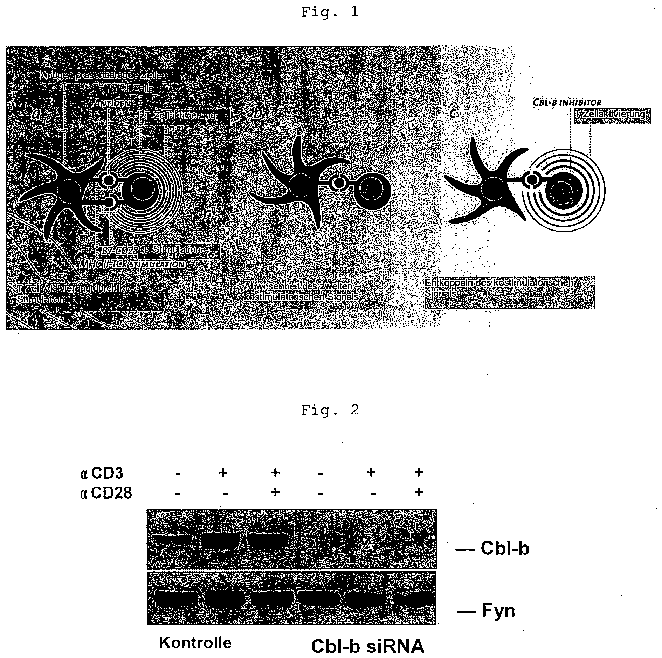

Mittels CPT Röhrchen (Vacutainer) wurde einem Spender Vollblut abgenommen und daraus PBMCs durch Zentrifugation abgetrennt. Aus dieser Präparation wurden in einem weiteren Schritt CD8⁺ Zellen angereichert. Diese wurden mittels einer Cbl-b spezifischen siRNA unter Verwendung eines Amaxa Transfektionsgerätes transfiziert (Detailprotokoll Beispiel 3) und weiter kultiviert. Als Kontrolle wurde der identische Ansatz mit einer unspezifischen siRNA unter Verwendung des identischen Protokolls transfiziert. Nachdem eine potentielle Überschneidung der Funktion von Cbl-b und c-Cbl vermutet wird, wurden zwei weitere Ansätze mittels c-Cbl spezifischer siRNA sowie einer Kombination aus c-Cbl und Cbl-b spezifischer siRNA behandelt. Alle Ansätze wurden für 2 Tage in Kultur genommen. Dass die Transfektion zur gewünschten Attenuierung der Cbl-b Expression geführt hat, wurde durch anschließende Western-Blot Analyse aufgezeigt. Um die Cbl-b Expression zu induzieren, wurden die Kulturen mit CD3 und in einem weiteren Ansatz mit CD3 und CD28 spezifischen Antikörpern stimuliert. Die Cbl-b Expression in der transfizierten Präparation wurde in allen Versuchen auf unter 5 % der Intensität einer Kontrolltransfektion gedrückt, wie in Fig. 2 ersichtlich ist. Nachdem die Stabilität der siRNA und demzufolge eine effiziente Expressionsunterdrückung von begrenzter Dauer ist und nicht an weitere Zellen weitergegeben wird, handelt es sich bei dem gewählten Ansatz um eine transiente Attenuierung der Cbl-b Expression, die strikt an die Anwesenheit der Cbl-b siRNA gebunden ist.

### Beispiel 3: Transfektionsprotokoll

### Nukleofektion mit siRNAs in humane T-Zellen

Nukleofektion wird zusammen mit einem anderen Labor-Angestellten durchgeführt. Eine Person übernimmt die Pipettierung der siRNA Oligos und ein anderer Angestellter transferiert die Proben in ein Nährmedium. Dies beschleunigt das Protokoll signifikant.
1. Stelle das Medium für die Zell-RPMI (+pen/strep., +L-glut, +10% FCS) her.
2. Pipettiere das Nährmedium zumindest in zwei 50 ml Falcon-Röhrchen/Konstrukt (eines zum Sammeln der Nukleofekt Zellen und eines für das Küvetten-Waschmedium), 1 ml/l Nukleofektions-Probe in jedes Röhrchen. Bringe die Röhrchen auf 37°C.
3. Markiere die Eppendorf-Röhrchen für jede Nukleofektions-Probe.
4. Zentrifugiere die Nukleofekt Zellen (410 g) für 7 min und entferne den Überstand. Füge Optimem (Gibco, #31985-047) hinzu,
5. so dass die Zelldichte 40 x 10⁶/ml beträgt. Pipettiere 100 µl (= 4 x 10⁶ Zellen) in jedes Eppendorf-Röhrchen.
6. Füge 1,5 - 2,5 µM siRNA-Oligo in die Eppendorf-Röhrchen hinzu, die die Zellen gerade vor der Nukleofektion enthalten. Mixen durch Pipettieren und übertrage die Lösung in die Küvette (vermeide Luftblasen). Klopfe die Küvette gegen den Tisch, um die Blasen zu entfernen.
7. Schließe den Deckel und stelle die Küvette in die Amaxa Transfektions-Vorrichtung (Elektroporator). (Programmiere U-14 und nicht V-24 als beste Option mit der Nukleofektor-Lösung Optimem). Drücke den x-Knopf und entferne die Küvette nach dem OK-Signal. (Drücke den x-Knopf vor der nächsten Elektroporation nochmals.)
8. Füge unverzüglich 500 µl RPMI (37°C) in die Nukleofektions-Küvette und mische behutsam durch Pipettieren mit der Pasteuer-Pipette. Übertrage die Zellen in das Sammel-Falcon-Röhrchen. Wasche die Küvette einmal mit 500 µl vorerwärmter RPMI und Übertragen den Rest der Zellen in das Sammel-Falcon-Röhrchen.
9. Wiederhole die Schritte 5-7 mit jeder Probe.
10.Stelle die Falcons in den Inkubator, bis alle Proben nukleofektiert sind.
11.Pipettiere 4 ml der Zellsuspension in 6-Well-Platten-Wells (= 2 Proben) und gibt die Platten in den Inkubator.
12.Sammle am nächsten Tag die Zellen, zähle sie und starte die Kultur 24 Stunden nach der wie oben beschrieben durchgeführten Nukleofektion. Nimm ein Aliquot der Zellen zur RNA-Isolierung, um zu überprüfen, dass das Target-Gen zum Zeitpunkt der Aktivieruung herunterreguliert ist. Die Proteinproben werden auf Grundlage der Protein-Expressions-Kinetik genommen.

### Beispiel 4: Nucleofektionseffizienz

CD8⁺ wurde w.o. beschrieben aus humanem peripherem Blut isoliert. Die neg. Selektion erfolgt über Beads.

### Resultat (Vergleich Amaxa):

**Reinheit der Population: 97% CD8⁺ (FACS)**

| Gerät | Viability nach 3,5h trypanblau negative [%] | Viability nach 24h trypanblau negative [%] | Effizienz siGlo pos im FACS [%] |
|---|---|---|---|
| Amaxa V-24 | 94 | 93 | 96 |
| unbehandelt | 100 | 99 | 0 |

**Versuch mit Optimem:**

| Nucleofection in | Programm | Zellen danach in | Viability nach 3,5h trypanblau negative [%] | Viability nach 24h trypanblau negative [%] | Effizienz siGlo pos im FACS [%] |
|---|---|---|---|---|---|
| Nucleofector Solution | V-24 | hTC | 99 | 92 | 96 |
| Optimem | V-24 | RPMI | 92 | 67 | 96 |
| Optimem | U-14 | RPMI | 95 | 92 | 96 |

Proteinchemisch wurde somit eine deutlich herabgesetzte Expression von Cbl-b nachgewiesen.

### Beispiel 5: Transiente Steigerung der T-Zell Reaktivität - Messung von IFN-gamma

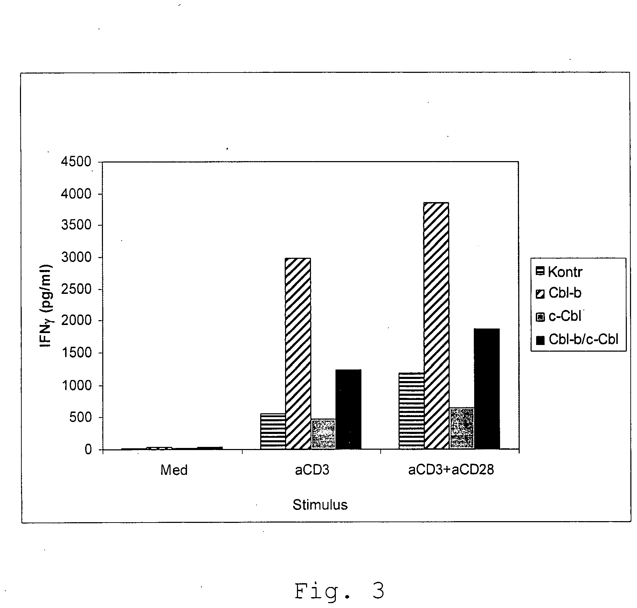

Es wurde somit gezeigt, dass die Cbl-b Expression in humanen CD8⁺ Zellen mit mindestens 95 % Effizienz unterdrückt werden konnte. Als weitere Konsequenz wird nun gezeigt, dass die Reaktivität der T-Zellpopulation gleichermaßen gesteigert werden kann. Um sicherzustellen, dass der gewünschte Effekt exklusiv Cbl-b spezifisch ist und nicht im Falle der Attenuierung der Cbl-b Expression durch c-Cbl umgangen werden kann, wurde in einem weiteren Ansatz ebenfalls c-Cbl und in einem dritten c-Cbl und Cbl-b attenuiert. All diese CD8⁺ Zellkulturen wurden für zwei Tage in Kultur gehalten und einerseits mittels CD3 und ferner mittels CD3 und CD28 stimuliert und mit einer nicht stimulierten Gruppe verglichen. Normalerweise benötigen T-Zellen diese Kostimulation von CD3 und CD28, um zu proliferieren, was durch eine Ausschüttung inflammatorischer Zytokine in den Überständen der Kulturen leicht nachgewiesen werden kann. Um diese T-Zellaktivierung zu erfassen wurden IFN-gamma Titer in den Überständen gemessen. Eine graphische Darstellung des Einflusses der Expressionsattenuierung durch siRNA Behandlung auf die T-Zellreaktivität ist in Fig. 3 wiedergegeben. 2 Tage nach der Transfektion wurden sämtliche CD8⁺ Zellkulturen, die mit Cbl-b und/oder c-Cbl spezifischer siRNA transfiziert wurden, wie beschrieben stimuliert und mit einer Kontrollgruppe, die mit einer nicht spezifischen siRNA transfiziert wurde, verglichen. Unstimulierte Zellen zeigten grundsätzlich keine IFN-gamma Expression auf. Nach exklusiver CD3 Stimulation zeigten alle Kulturen eine erhöhte Reaktivität, so dass mindestens 500 pg/ml IFN-gamma in den Überständen nachgewiesen werden konnte. Hierbei waren die Signale für c-Cbl spezifische siRNA und unspezifisch transfizierte Zellen sehr ähnlich (niedrig). Lediglich mit Cbl-b spezifischer siRNA transfizierte Zellen zeigten eine deutlich höhere Reaktivität, welche mit einem IFN-gamma Titer von ca. 3 ng/ml einherging. Die Reaktivität der mittels c-Cbl und Cbl-b spezifischer siRNA ko-transfizierten Zellpräparation war jedoch geringer als nach Cbl-b spezifischer siRNA Behandlung und erreichte nur 1,2 ng/ml. Eine CD3 und CD28 spezifische Kostimulation erbrachte, wie erwartet, in allen Fällen deutlich höhere Signale als die nur CD3 spezifische Stimulation. Die Kontrollpräparation, die mit unspezifischer siRNA behandelt wurde, erbrachte IFN-gamma Titer in einer Höhe von 1,2 ng/ml während die mittels Cbl-b spezifischer siRNA behandelte Kultur eine IFN-gamma Konzentration um 3,8 ng/ml aufwies. Bemerkenswert war, dass die Cbl-b und c-Cbl spezifisch koattenuierte Kultur Titer in der Höhe von 1.7 ng/ml aufzeigte, die demnach deutlich geringer waren als die in der Cbl-b attenuierten Gruppe. Unerwartet war auch, dass die Zellpopulation, die mit c-Cbl spezifischer siRNA behandelt wurde, eine markant geringere Reaktivität aufwies und nur 500 pg/ml IFN-gamma im Überstand gemessen wurden. Diese Konzentration ist deutlich geringer als die der unspezifisch behandelten ko-stimulierten Kontrollgruppe und vergleichbar mit Werten der nur mit anti-CD3 stimulierten Kontrollgruppe. Damit ist im Gegensatz zum murinen System eine Redundanz von Cbl-b and c-Cbl in humanen CD8⁺ T-Zellen experimentell ausgeschlossen und ein therapeutischer Ansatz nur über Cbl-b (und dessen Upstream-regulators) aber nicht via Cbl-b/c-Cbl Kombinationen zur Erhöhung der Immunreaktivität sinnvoll. Die Inhibition von c-Cbl ermöglicht allerdings eine Immunsuppression für andere Anwendungen, wie z.B. die Behandlung von Inflammation oder Allergien.

Dieser entweder als Solotherapie konzipierte oder mit einer Vakzinierung einhergehende, begleitende therapeutische Ansatz ist ferner in der Lage, disseminierte Tumorzellen auch in der Peripherie zu erkennen und durch den Aufbau einer Immunantwort nachhaltig zu bekämpfen. Sofort nach Diagnose einer Krebserkrankung eingesetzt wird hiermit ebenfalls eine Disseminierung eines Primärtumors verhindert.

### Beispiel 6: Transiente Steigerung der T-Zell Reaktivität - Messung von IL2

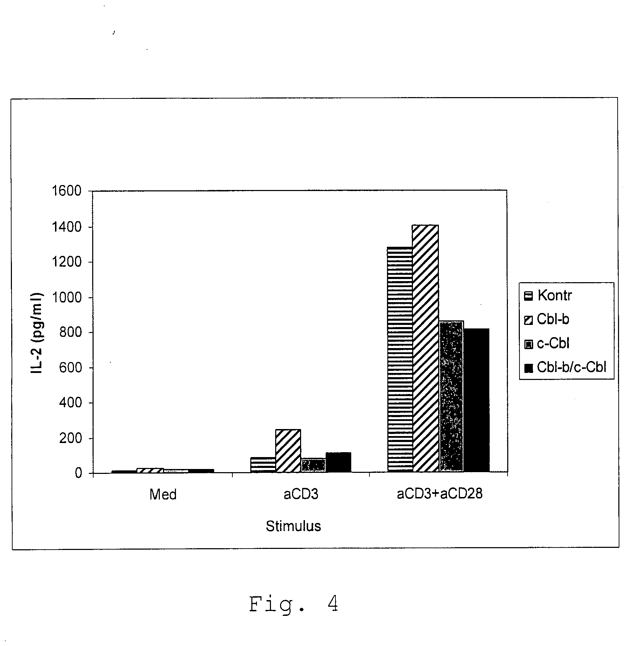

Ein sehr ähnliches Bild ergab sich durch Messung der IL2 Konzentrationen in denselben Überständen, wie aus Fig. 4 entnommen werden kann: Ohne Stimulation gab es keine messbare Antwort, während eine anti-CD3 spezifische Behandlung zu einem deutlichen messbaren Signal in allen Gruppen führte. Somit wurden >200 pg/ml in der Cbl-b attenuierten Gruppe gemessen. Die IL2 Konzentration in der Cbl-b und c-Cbl koattenuierten Population war wiederum deutlich niedriger und betrug <100 pg/ml. Die anti-CD3 und anti-CD28 spezifische Kostimulation ergab wiederum höhere Signale. Es wurden in allen Gruppen unabhängig von der siRNA Behandlung IL2 Konzentrationen von >800 pg/ml gemessen. Die Kontrollgruppe erbrachte sehr ähnliche Titer wie die Cbl-b attenuierte von 1,3 bzw. 1,4 ng/ml. Interessanterweise waren die IL2 Konzentrationen nach c-Cbl spezifischer Attenuierung deutlich geringer und betrugen nur 800 pg/ml unabhängig davon, ob nur c-Cbl exklusiv oder c-Cbl zusammen mit Cbl-b im Ansatz verwendet wurden.

### Beispiel 7: Transiente Steigerung der Immunreaktivität

Durch die in vitro oder ex vivo Behandlung wird die T-Zell-Reaktivität effizient gesteigert, so dass es durch alleinige Stimulation des T-Zellrezeptors möglich ist, die Proliferation der T-Zellen einzuleiten. Dies ist eine wesentliche Voraussetzung für den beschriebenen therapeutischen Ansatz. Aufgrund der Verwendung der RNAi Technologie ist diese Attenuierung transienter Natur.

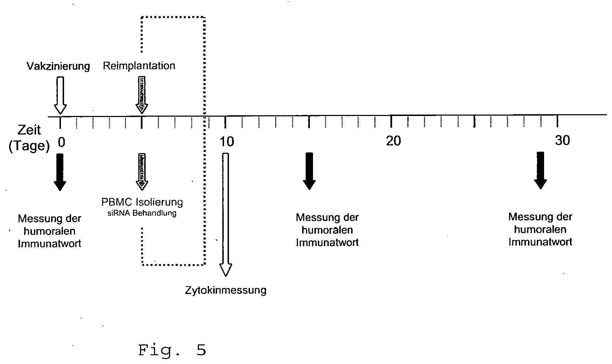

Diese Anwendbarkeit dieser modifizierten Zellen dient der Steigerung der Immunogenität von Vakzinen sowie zur allgemeinen Erhöhung der Reaktivität des Immunsystems. Einem Patienten wird fünf Tage nach einer Grundimmunisierung Vollblut in CPT Röhrchen abgenommen. Nach ca. 20 Minuten werden daraus durch Zentrifugation PBMCs isoliert. Die Zellpräparationen werden mit Cbl-b spezifischen siRNAs transfiziert und sofort danach dem Patienten wieder re-implantiert. Zum Tag 10 wird wieder Vollblut entnommen und daraus Serum gewonnen. In diesem werden pro-inflammatorische Zytokintiter gemessen und mit der Kontrollgruppe verglichen. Es wird ebenfalls die Natur der induzierten Immunantwort hinsichtlich deren zellulärer Ausrichtung einer Th1 gesteuerten (gesteigerte IFN-gamma, IL2 und IL12 Titer) oder einer humoralen Tendenz einer Th2 gerichteten Immunantwort (gesteigerte IL4, IL5 und IL10 Titer) analysiert. Falls nötig werden weitere Boosterimmunisierungen im 14 Tagesintervall mit oder ohne PBMC Behandlung durchgeführt (Fig. 5).

### Beispiel 8: Nucleofektion von CD4 T-Zellen und CD19 B-Zellen

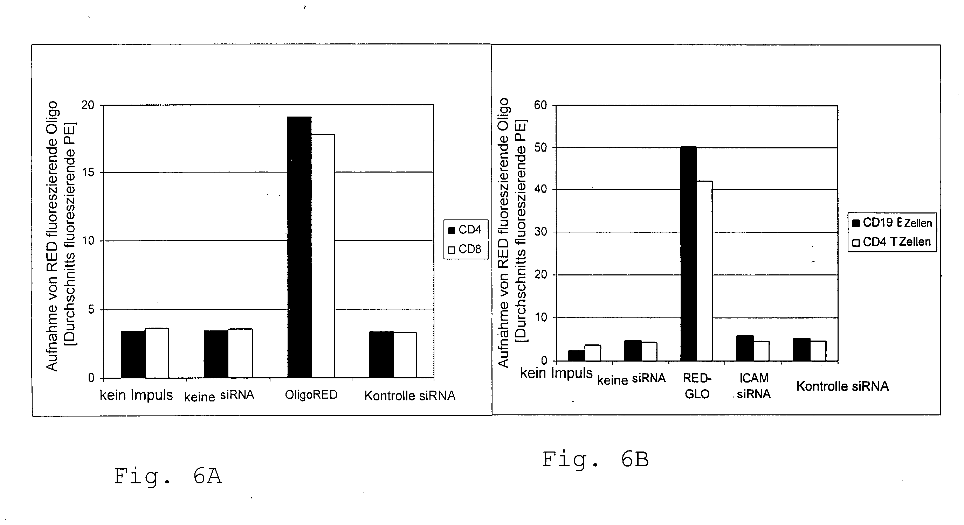

PBMCs wurden wie oben beschrieben isoliert und unter den selben Bedingungen wie in Beispiel 3 (U14) transfiziert. Das zur Transfektion verwendete Oligo (siGLO Red) wurde in einer Konzentration von 2µM eingesetzt und die spezifische Aufnahme mittels FACS nachgewiesen, die Unterscheidung von CD4 und CD8 Zellen wurde durch gleichzeitige Doppelfärbung mit CD8-FITC und CD3-APC durchgeführt (Fig. 6A).

PBMCs wurden wie oben beschrieben präpariert und deren CD8-Zellen isoliert. Die restlichen CD8-negativen Zellen wurden dann wieder wie oben mit siGLO Red transifiziert (allerdings mit einer Oligokonzentration von 3,3 µM). Die spezifische Aufnahme wurde mittels FACS durch gleichzeitige Doppelfärbung mit CD3-FITC und CD19-APC nachgewiesen (Fig. 6B). Interessanterweise wurde in diesem Experiment auch beobachtet, dass in der Fraktion der CD3/CD19-negativen Lymphozyten - welche hauptsächlich aus NK-Zellen besteht - ebenso eine effiziente Aufnahme des verwendeten Oligos stattfand.

Dieses Beispiel zeigt daher, dass auch andere Immunzellen unter denselben Transfektionsbedingungen wie CD8-Zellen hocheffizient transfiziert werden können.

### Beispiel 9: Transiente Reduktion der Cbl-b Expression in humanen CD4-Zellen auf mRNA und Protein-Ebene

CD4-Zellen wurden aus PBMCs durch Depletion von CD8-Zellen isoliert und mittels PHA/anti-CD3/28-Stimulation kultiviert. Nach 2 Wochen wurden diese CD4 zellen mittels einer Cbl-b spezifischen siRNA unter Verwendung des Amaxa Transfektionsprotokolls transfiziert (siehe Beispiel 3 & 8). Als Kontrolle wurde der identische Ansatz mit einer unspezifischen siRNA unter Verwendung des identischen Protokolls transfiziert. Nach der Transfektion wurden die Zellen mit IL-2 (5ng/ml) für einen weiteren Tag kultiviert und am nächsten Tag mit anti-CD3/28 Antikörpern stimuliert.

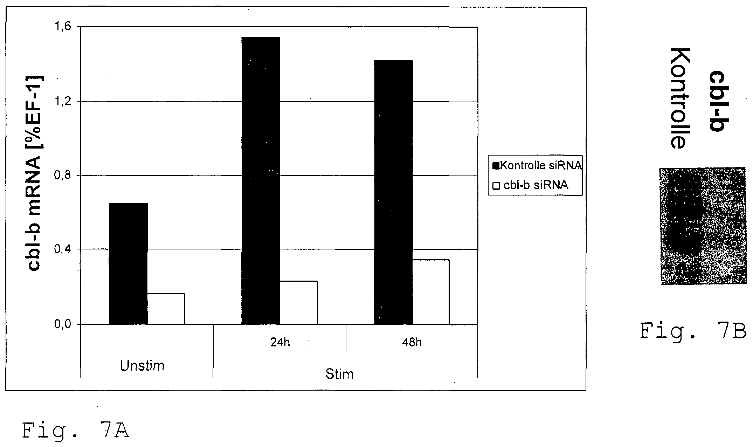

Die Cbl-b mRNA Expression in der transfizierten Präparation wurde in den für 24h stimulierten CD4-Zellen um ca. 85% im Vergleich zur Kontrolltransfektion verringert (Fig. 7A).
Die starke Verringerung der Cbl-b mRNA korrelierte dabei mit einer vergleichsweise starken Verringerung der Proteinmenge von Cbl-b, wie im Western blot nachgewiesen wurde (Fig. 7 B).

### Beispiel 10: Steigerung der CD4 T-Zell Reaktivität - Messung von Zytokinen mit Anti-Tumoraktivität

Eine der Hauptaufgaben von CD4-Zellen in T-Zell vermittelten Immunantworten ist die Produktion von inflammatorischen Zytokinen. Als für die Anti-Tumoraktivität insbesonders von Bedeutung werden in der Literatur die Zytokine IL-2, IFN-γ und TNF-α genannt.

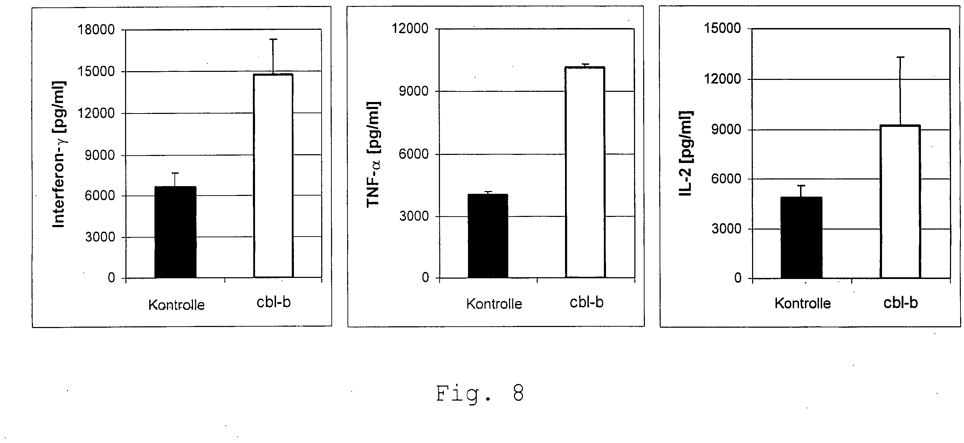

Die Expression von diesen drei Zytokinen wurde daher mittels ELISA bestimmt. Zum Zeitpunkt 24h nach anti-CD3/28-Stimulation waren diese drei Zytokine in humanen Cbl-b gesilenceden CD4 T-Zellen signifikant erhöht (Fig. 8).

### Beispiel 11: Transienter Verlauf der Steigerung der CD4 T-Zell Reaktivität durch vermehrte Produktion von Zytokinen mit Anti-Tumoraktivität

Um eine effiziente Anti-Tumoraktivität von Cbl-b gesilenceden T-Zellen zu erreichen ist es wichtig, dass die vermehrte Zytokin-Produktion auch über einen gewissen Zeitraum nach T-Zell-Stimulation aufrechterhalten bleibt. Allerdings sollte dieser Zeitraum auch begrenzt sein um das Risiko einer dauerhaften Etablierung von unerwünschter Autoimmunität gegen nicht-malignes Gewebe des Patienten zu minimieren.

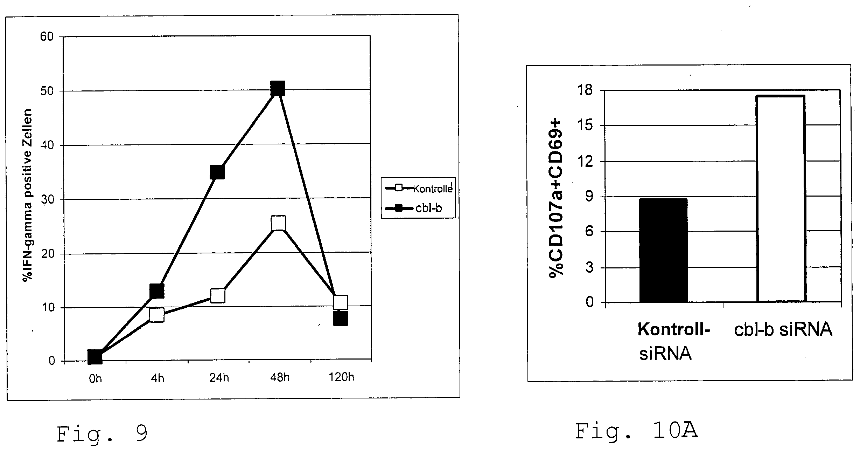

Es wurde daher die Produktion von IFN-γ auch zu verschiedenen Zeitpunkten mittels intrazellulärem Staining im FACS analysiert. Die Abbildung zu Fig. 9 zeigt klar, dass die starke Erhöhung der IFN-γ Produktion über zumindest 48h bestehen blieb, während sie 6 Tage nach Stimulation wieder auf einen vergleichbaren Wert wie in der Kontrolle zurückfiel.

### Beispiel 12: Steigerung der T-Zell Reaktivität - erhöhte Expression von Oberflächenmolekülen mit funktionellen Eigenschaften bzw. Stimulationsmarkerfunktion.

Die jeweilige Bestimmung der Zytokinproduktion als Marker für funktionell erfolgreiches Silencing von Cbl-b in humanen T-Zellen ist technisch aufwändiger und kann daher tendenziell nicht so zeitnah durchgeführt werden. Es wurde daher auch die Expression funktionell wichtiger Oberflächenmarker mittels FACS bestimmt.

CD107a ist in der Literatur definiert als Oberflächenmarker für sekretorische Aktivität zytotoxischer T-Zellen, und wurde daher an Cbl-b unterdrückten CD8 T-Zellen nach 24h anti-CD3/28 Stimulation bestimmt. Die mit Cbl-b siRNA transfizierten T-Zellen zeigten dabei eine wesentlich erhöhte sekretorische Aktivität (Fig. 10A).

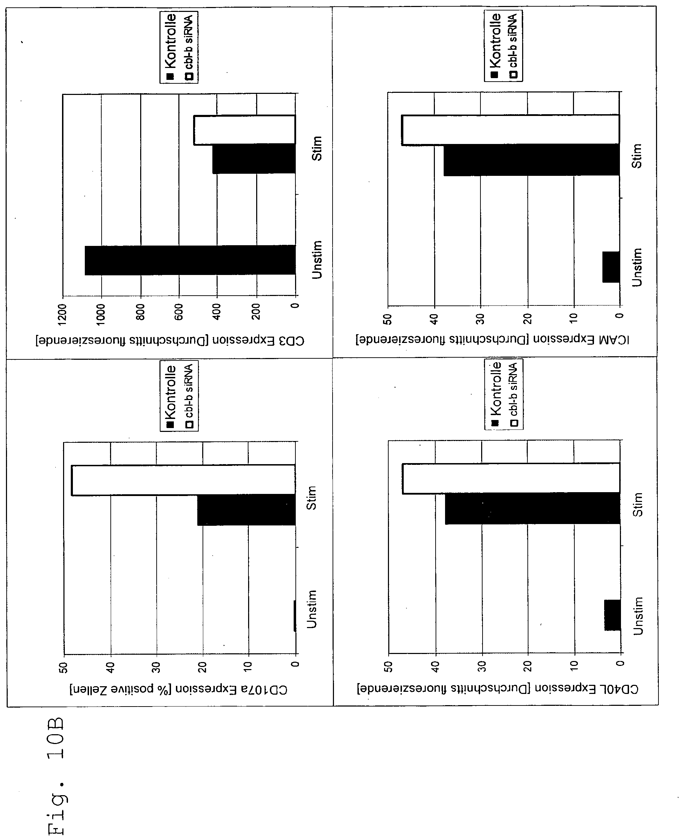

Da das CD107a Molekül am vesikulären Transport in T-Zellen beteiligt ist kann es auch als prinzipieller Marker für die sekretorische Aktivität von CD4 T-Zellen verwendet werden. Fig. 10B zeigt, dass die Expression von CD107a auch in Cbl-b siRNA transfizierten Zellen signifikant gegenüber ansonsten gleich behandelten Kontrollzellen war.

Zwei weitere durch T-Zell-Stimulation induzierbare Oberflächenmoleküle sind CD40L und ICAM. Beide Moleküle sind von funktioneller Relevanz, CD40L vor allem für die Interaktion mit Antigen-präsentierenden Zellen und für die Stimulation/Proliferation von B-Zellen, ICAM für die Interaktion mit Antigen-präsentierenden Zellen und die Migration aus dem Gefäßsystem ins (maligne) Gewebe. Fig. 10B zeigt, dass in Cbl-b siRNA transfizierten humanen CD4 T-Zellen die Expression dieser beiden Oberflächenmoleküle signifikant erhöht war.

Einer der beschriebenen Mechanismen verantwortlich für die Erhöhung der T-Zell-Reaktivität ist die weniger stark ausgeprägte Attenuierung von an der Zelloberfläche befindlichen CD3-Rezeptoren. Fig. 10B zeigt, dass auch in Cbl-b siRNA transfizierten humanen CD4 T-Zellen die Menge an noch auf der Zelloberfläche befindlichen CD3-Rezeptoren eindeutig erhöht war.

Interessanterweise war auch die Zelloberflächenexpression des klassischen T-Zellaktivierungsmarkers CD69 erhöht, während die Expression von CD25 unverändert blieb. Dies mag von besonderer funktioneller Relvanz sein, denn obwohl CD25 auch als Stimulationsmarker definiert ist, so ist seine Funktion besonders mit dem Vorhandensein und Überleben von sogenannten T-regulatorischen Zellen assoziiert.

Insgesamt zeigt Figur 10 daher, dass weitere funktionell bedeutsame oder als Oberflächenmarker dienliche Moleküle durch Cbl-b siRNA Transfektion in eindeutig höheren Mengen auf der Zelloberfläche nachgewiesen werden können.

### Beispiel 13: Der gemeinsame Transfer von Cbl-b defizienten T-Zellen und dendritischen Zellen ist ein wirksames Therapieverfahren in einem in vivo Tumormodell.

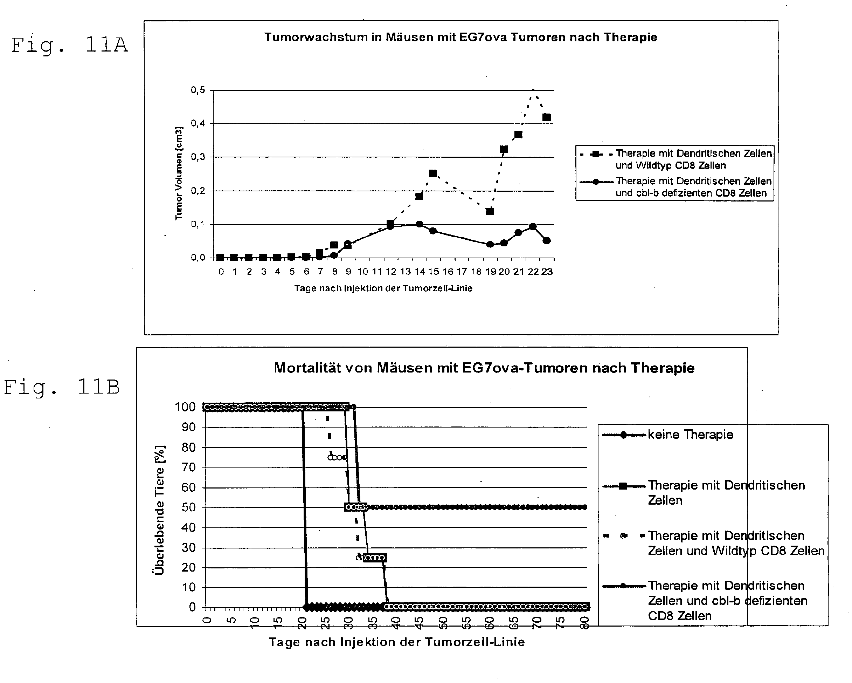

In Wildtypmäusen wurde durch subkutane Injektion von 0,1 Millionen EG7ova-Zellen ein Tumor induziert. Am Tag 5 und 6 wurden dann CD8 T-Zellen und dendritische Zellen injiziert und der Effekt dieser adoptiven Zelltherapie durch Abmessung des Tumorwachstums stetig verfolgt. Fig. 11A zeigt, dass das Tumorwachstum durch den Transfer von Cbl-b defizienten T-Zellen wesentlich stärker und nachhaltiger unterdrückt werden konnte als durch Wildtyp T-Zellen.

Fig. 11B zeigt darüberhinaus, dass die Therapie mit Cbl-b defizienten T-Zellen ein langfristiges Überleben eines signifikanten Teils der behandelten Mäuse bis zum Ende des Beobachtungszeitraumes von 80 Tagen gewährleisten konnte. Im Gegensatz dazu führte die Behandlung mit Wildtyp T-Zellen nur zu einer geringfügigen Lebensverlängerung im Vergleich zu den Kontrollgruppen. Fig. 11B zeigt daher, dass eine Therapie mit Cbl-b defizienten oder inhibierten T-Zellen einen signifikanten Vorteil gegenüber einer Therapie mit normalen T-Zellen hat.

## Patentansprüche

1. In vitro oder ex vivo Verfahren zur Erhöhung der Immunreaktivität von Zellen des Immunsystems, umfassend die transiente Reduzierung oder Inhibierung der Expression von Cbl-b durch Verwendung einer kurzen DNA und/oder RNA Sequenz komplementär zu einem Teil der Ziel-Cbl-b-mRNA Sequenz, wobei die Zellen vor oder nach der Entnahme mit einem Antigen kontaktiert wurden, und wobei die Immunreaktivität der Zellen gegen das Antigen erhöht wird, und wobei die Zellen antigenpräsentierende Zellen, PBMCs (Peripheral Blood Mononuclear Cells), T-Lymphozyten, B-Lymphozyten, Monozyten, Makrophagen, NK Zellen und/oder NKT Zellen umfassen.

2. Verfahren nach Anspruch 1, wobei die T-Lymphozyten CD8+ T-Lymphozyten und/oder CD4+ T-Lymphozyten umfassen.

3. Verfahren nach Anspruch 2, wobei die CD8+ T-Lymphozyten regulatorische T Zellen (Tregs) und/oder zytotoxische T-Zellen (CTLs) umfassen.

4. Verfahren nach Anspruch 2 oder 3, wobei die CD4+ T-Lymphozyten Th1, Th2, und/oder Th17 umfassen.

5. Verfahren nach einem der Ansprüche 1 bis 4, **dadurch gekennzeichnet, dass** die Zellen das Antigen aufgenommen haben und vorzugsweise ein Antigenfragment präsentieren.

6. Verfahren nach einem der Ansprüche 1 bis 5, **dadurch gekennzeichnet, dass** die Zellen mit einer immunstimulierenden Substanz, vorzugsweise einem Cytokin, behandelt werden.

7. Pharmazeutische Zusammensetzung umfassend eine kurze DNA und/oder RNA Sequenz komplementär zu einem Teil der Ziel-Cbl-b-mRNA Sequenz zur Anwendung in einer Therapie umfassend Isolierung von Zellen des Immunsystems eines Patienten, wobei die Zellen antigenpräsentierende Zellen, PBMCs (Peripheral Blood Mononuclear Cells), T-Lymphozyten, B-Lymphozyten, Monozyten, Makrophagen, NK Zellen und/oder NKT Zellen umfassen; in vitro oder ex vivo Erhöhung der Immunreaktivität der Zellen durch Verwendung der kurzen DNA und/oder RNA Sequenz komplementär zu einem Teil der Ziel-Cbl-b-mRNA Sequenz, vorzugsweise wie in einem der Ansprüche 1 bis 6 definiert, und Reimplantation der Zellen in den Patienten, wobei durch eine transiente Reduktion oder die Inhibition der Cbl-b Funktion der Zellen die Immunreaktivität erhöht wird.

8. Isolierte Zellen des Immunsystems eines Patienten, die mit einer kurzen DNA und/oder RNA Sequenz komplementär zu einem Teil der Ziel-Cbl-b-mRNA Sequenz transient transfiziert wurden, wobei die Zellen antigenpräsentierende Zellen, PBMCs (Peripheral Blood Mononuclear Cells), T-Lymphozyten, B-Lymphozyten, Monozyten, Makrophagen, NK Zellen und/oder NKT Zellen umfassen; und wobei die Zellen vor oder nach der Entnahme mit einem Antigen kontaktiert wurden, zur Anwendung in einer Therapie umfassend die Reimplantation der Zellen in den Patienten, wobei durch eine transiente Reduktion oder die Inhibition der Cbl-b Funktion der Zellen die Immunreaktivität erhöht wird, vorzugsweise wie in einem der Ansprüche 1 bis 6 definiert.

9. Pharmazeutische Zusammensetzung zur Anwendung nach Anspruch 7 oder isolierte Zellen zur Anwendung nach Anspruch 8 zur Behandlung einer angeborenen oder erworbenen Immuninsuffizienz, insbesondere AIDS, multiples Myelom, chronische lymphatische Leukämie, medikamentenbedingte Immunsuppression oder einer Krebserkrankung, vorzugsweise eine Krebserkrankung, die feste Tumore bildet, insbesondere zur Behandlung einer Krebserkrankung in Kombination mit einer weiteren Anti-Tumor Therapie, vorzugsweise Chemotherapie, Radiotherapie, Verabreichung eines Biologics oder Dentritische-Zellen gestützter Vakzinierung, insbesondere einer Tumorvakzinierung.

10. Pharmazeutische Zusammensetzung oder isolierte Zellen zur Anwendung nach einem der Ansprüche 7 bis 9, wobei die T-Lymphozyten CD8+ T-Lymphozyten, und/oder CD4+ T-Lymphozyten umfassen..

11. Pharmazeutische Zusammensetzung oder isolierte Zellen zur Anwendung nach Anspruch 10, wobei die CD8+ T-Lymphozyten regulatorische T Zellen (Tregs) und/oder zytotoxische T-Zellen (CTLs) umfassen.

12. Pharmazeutische Zusammensetzung oder isolierte Zellen zur Anwendung nach Anspruch 10 oder 11, wobei die CD4+ T-Lymphozyten Th1, Th2, und/oder Th17 umfassen.

13. Pharmazeutische Zusammensetzung oder isolierte Zellen zur Anwendung nach einem der Ansprüche 7 bis 12, **dadurch gekennzeichnet, dass** die Zellen mit einer immunstimulierenden Substanz, vorzugsweise einem Cytokin, behandelt werden.

14. Pharmazeutische Zusammensetzung oder isolierte Zellen zur Anwendung nach einem der Ansprüche 7 bis 13, **dadurch gekennzeichnet, dass** der Patient mit dem Antigen beimpft wurde, vorzugsweise vor der Isolierung der Zellen, insbesondere bevorzugt vor mindestens 2 Tagen und/oder höchstens 8 Wochen vor Isolierung der Zellen.

15. Verfahren nach einem der Ansprüche 1 bis 6, oder pharmazeutisehe Zusammensetzung oder isolierte Zellen zur Anwendung nach einem der Ansprüche 7 bis 14, **dadurch gekennzeichnet, dass** die Zellen in vitro oder ex vivo mit dem Antigen kontaktiert werden.

16. Verfahren nach einem der Ansprüche 1 bis 6 und 15, oder pharmazeutische Zusammensetzung oder isolierte Zellen zur Anwendung nach einem der Ansprüche 7 bis 15, **dadurch gekennzeichnet, dass** Zellen spezifisch für ein bestimmtes Antigen oder Zellen umfassend ein bestimmtes Antigen auf die Spezifität oder Anwesenheit des Antigens selektioniert werden und die Immunreaktivität der selektionierten Zellen erhöht wird, wobei vorzugsweise das Antigen ein Tumor Antigen ist.

17. Verfahren nach einem der Ansprüche 1 bis 6 und 15 oder 16, oder pharmazeutische Zusammensetzung oder isolierte Zellen zur Anwendung nach einem der Ansprüche 7 bis 16, **dadurch gekennzeichnet, dass** die Zellen vor der Reimplantation expandiert werden.

## Claims

1. An in vitro or ex vivo method of increasing the immunoreactivity of cells of the immune system including the transient reduction or inhibition of the expression of Cbl-b by using a short DNA and/or RNA sequence complementary to a portion of the target CB1-b-mRNA sequence, wherein the cells were contacted before or after the removal with an antigen and wherein the immunoreactivity of the cells to the antigen is increased and wherein the cells include antigen-presenting cells, PBMCs (Peripheral Blood Mononuclear Cells), T lymphocytes, B lymphocytes, monocytes, macrophages, NK cells and/or NKT cells.

2. A method as claimed in Claim 1, wherein the T lymphocytes include CD8+ T lymphocytes and/or CD4+ T lymphocytes.

3. A method as claimed in Claim 2, wherein the CD8+ T lymphocytes include regulatory T cells (Tregs) and/or cytotoxic T cells (CTLs).

4. A method as claimed in Claim 2 or 3, wherein the CD4+ T lymphocytes include Th1, Th2 and/or TH17.

5. A method as claimed in one of Claims 1 to 4, **characterised in that** the cells have absorbed the antigen and preferably present an antigen fragment.

6. A method as claimed in one of Claims 1 to 5, **characterised in that** the cells are treated with an immunostimulating substance, preferably a cytokine.

7. A pharmaceutical composition including a short DNA and/or RNA sequence complementary to a portion of the target Cb1-b-mRNA sequence for use in a therapy including isolation of cells of the immune system of a patient, wherein the cells include antigen-presenting cells, PBMCs (Peripheral Blood Mononuclear Cells), T lymphocytes, B lymphocytes, monocytes, macrophages, NK cells and/or NKT cells; in vitro or ex vivo increase of the immunoreactivity of the cells by using the short DNA and/or RNA sequence complementary to a portion of the target Cb1-b-mRNA sequence, preferably as defined in one of Claims 1 to 6, and reimplantation of the cells into the patient, wherein the immunoreactivity is increased by a transient reduction or the inhibition of the Cbl-b function of the cells.

8. Isolated cells of the immune system of a patient, which were transiently transfected with a short DNA and/or RNA sequence complementary to a portion of the target Cb1-b-mRNA sequence, wherein the cells include antigen-presenting cells, PBMCs (Peripheral Blood Mononuclear Cells), T lymphocytes, B lymphocytes, monocytes, macrophages, NK cells and/or NKT cells; and wherein the cells were contacted before or after the removal with an antigen, for use in a therapy including the reimplantation of the cells into the patient, wherein as a result of the transient reduction or the inhibition of the Cbl-b function of the cells the immunoreactivity is increased, preferably as defined in one of Claims 1 to 6.

9. A pharmaceutical composition for use as claimed in Claim 7 or isolated cells for use as claimed in Claim 8 for treating a congenital or acquired immunodeficiency, particularly AIDS, multiple myeloma, chronic lymphatic leukaemia, medicinally caused immunosuppression or a cancerous disease, preferably a cancerous disease which forms solid tumours, particularly for treating a cancerous illness in combination with a further anti-tumour therapy, preferably chemotherapy, radiotherapy, administration of a biological preparation or dendritic cell based vaccination, particularly a tumour vaccination.

10. A pharmaceutical composition or isolated cells for use as claimed in one of Claims 7 to 9, wherein the T lymphocytes include CD8+ T lymphocytes and/or CD4+ T lymphocytes.

11. A pharmaceutical composition or isolated cells for use as claimed in Claim 10, wherein the CD8+ T lymphocytes include regulatory T cells (Tregs) and/or cytotoxic T cells (CTLs).

12. A pharmaceutical composition or isolated cells for use as claimed in Claim 10 or 11, wherein the CD4+ T lymphocytes include Th1, Th2 and/or TH17.

13. A pharmaceutical composition or isolated cells for use as claimed in one of Claims 7 to 12, **characterised in that** the cells are treated with an immunostimulated substance, preferably a cytokine.

14. A pharmaceutical composition or isolated cells for use as claimed in one of Claims 7 to 13, **characterised in that** the patient was inoculated with the antigen, preferably before the isolation of the cells, particularly preferably before at least 2 days and/or at most 8 weeks before isolation of the cells.

15. A method as claimed in one of Claims 1 to 6 or a pharmaceutical composition or isolated cells for use as claimed in one of Claims 7 to 14, **characterised in that** the cells are contacted in vitro or ex vivo with the antigen.

16. A method as claimed in one of Claims 1 to 6 and 15 or a pharmaceutical composition or isolated cells for use as claimed in one of Claims 7 to 15, **characterised in that** cells specific for a certain antigen or cells including a certain antigen are selected with regard to the specificity or presence of the antigen and the immunoreactivity of the selected cells is increased, wherein the antigen is preferably a tumour antigen.

17. A method as claimed in one of Claims 1 to 6 and 15 or 16 or a pharmaceutical composition or isolated cells for use as claimed in one of Claims 7 to 16, **characterised in that** the cells are expanded before the reimplantation.

## Revendications

1. Procédé in vitro ou ex vivo destiné à augmenter l'immunoréactivité de cellules du système immunitaire, comprenant la réduction transitoire ou l'inhibition de l'expression de Cb1-b par utilisation d'une séquence courte d'ADN et/ou d'ARN, complémentaire d'une partie de la séquence de l'ARNm de Cb1-b cible, les cellules ayant été, avant ou après leur prélèvement, mises en contact avec un antigène, et ce à l'occasion de quoi on a une augmentation de l'immunoréactivité des cellules vis-à-vis de l'antigène, et les cellules comprenant des cellules présentant l'antigène, des PBMC (cellules mononucléaires du sang périphérique), des lymphocytes T, des lymphocytes B, des monocytes, des macrophages, des cellules NK et/ou des cellules NKT.

2. Procédé selon la revendication 1, dans lequel les lymphocytes T comprennent des lymphocytes T CD8+ et/ou des lymphocytes T CD4+.

3. Procédé selon la revendication 2, dans lequel les lymphocytes T CD8+ comprennent des cellules T régulatrices (Treg) et/ou des cellules T cytotoxiques (CTL).

4. Procédé selon la revendication 2 ou 3, dans lequel les lymphocytes T CD4+ comprennent le Th1, le Th2 et/ou le Th17.

5. Procédé selon l'une des revendications 1 à 4, **caractérisé en ce que** les cellules ont absorbé l'antigène, et de préférence présentent un fragment d'antigène.

6. Procédé selon l'une des revendications 1 à 5, **caractérisé en ce que** les cellules sont traitées avec une substance immunostimulante, de préférence une cytokine.

7. Composition pharmaceutique comprenant une séquence courte d'ADN et/ou d'ARN complémentaire d'une partie de la séquence d'ARNm de Cb1-b cible, pour une utilisation dans une thérapie comprenant l'isolement de cellules du système immunitaire d'un patient, les cellules comprenant des cellules présentant l'antigène, des PBMC (Cellules Mononucléaires du Sang Périphérique), des lymphocytes T, des lymphocytes B, des monocytes, des macrophages, des cellules NK et/ou des cellules NKT ; l'augmentation in vitro ou ex vivo de l'immunoréactivité des cellules par utilisation de la séquence courte d'ADN et/ou d'ARN complémentaire d'une partie de la séquence d'ARNm de Cb1-b cible, de préférence comme défini dans l'une des revendications 1 à 6, et réimplantation des cellules dans le patient, une réduction transitoire ou l'inhibition de la fonction Cb1-b des cellules augmentant l'immunoréactivité.

8. Cellules isolées du système immunitaire d'un patient, qui ont subi une transfection transitoire avec une séquence courte d'ADN et/ou d'ARN complémentaire d'une partie de la séquence d'ARNm de Cb1-b cible, les cellules comprenant des cellules présentant l'antigène, des PBMC (Cellules Mononucléaires du Sang Périphérique), des lymphocytes T, des lymphocytes B, des monocytes, des macrophages, des cellules NK et/ou des cellules NKT, les cellules étant, avant ou après leur prélèvement, mises en contact avec un antigène, pour une utilisation dans une thérapie comprenant la réimplantation des cellules dans le patient, une réduction transitoire ou l'inhibition de la fonction Cb1-b des cellules augmentant l'immunoréactivité, de préférence comme défini dans l'une des revendications 1 à 6.

9. Composition pharmaceutique pour l'utilisation selon la revendication 7, ou cellules isolées pour l'utilisation selon la revendication 8, pour le traitement d'une insuffisance immunitaire innée ou acquise, de préférence d'un SIDA, d'un myélome multiple, d'une leucémie lymphatique chronique, d'une immunosuppression médicamenteuse ou d'une maladie cancéreuse, de préférence une maladie cancéreuse formant des tumeurs solides, en particulier pour le traitement d'une maladie cancéreuse en association avec une thérapie antitumorale supplémentaire, de préférence une chimiothérapie, une radiothérapie, une administration d'une substance biologique ou une vaccination assistée par des cellules dendritiques, en particulier une vaccination tumorale.

10. Composition pharmaceutique ou cellules isolées pour l'utilisation selon l'une des revendications 7 à 9, les lymphocytes T comprenant des lymphocytes T CD8+ et/ou des lymphocytes T CD4+.

11. Composition pharmaceutique ou cellules isolées pour l'utilisation selon la revendication 10, les lymphocytes T CD8+ comprenant des cellules T régulatrices (Treg) et/ou des cellules T cytotoxiques (CTL).

12. Composition pharmaceutique ou cellules isolées pour l'utilisation selon la revendication 10 ou 11, les lymphocytes T CD4+ comprenant le Th1, le Th2 et/ou le Th17.

13. Composition pharmaceutique ou cellules isolées pour l'utilisation selon l'une des revendications 7 à 12, **caractérisées en ce que** les cellules sont traitées avec une substance immunostimulante, de préférence une cytokine.

14. Composition pharmaceutique ou cellules isolées pour l'utilisation selon l'une des revendications 7 à 13, **caractérisées en ce que** le patient reçoit une inoculation de l'antigène, de préférence avant l'isolement des cellules, d'une manière particulièrement préférée au moins 2 jours et au plus 8 semaines avant l'isolement des cellules.

15. Procédé selon l'une des revendications 1 à 6 ou composition pharmaceutique ou cellules isolées pour l'utilisation selon l'une des revendications 7 à 14, **caractérisés en ce que** les cellules sont mises en contact in vitro ou ex vivo avec l'antigène.

16. Procédé selon l'une des revendications 1 à 6 et 15, ou composition pharmaceutique ou cellules isolées pour l'utilisation selon l'une des revendications 7 à 15, **caractérisés en ce qu'**on sélectionne les cellules spécifiquement pour un certain antigène ou les cellules comprenant un certain antigène, pour ce qui est de la spécificité ou de la présence de l'antigène, et on augmente l'immunoréactivité des cellules sélectionnées, l'antigène étant de préférence un antigène tumoral.

17. Procédé selon l'une des revendications 1 à 6 et 15 ou 16, ou composition pharmaceutique ou cellules isolées pour l'utilisation selon l'une des revendications 7 à 16, **caractérisés en ce que** les cellules sont expansées avant la réimplantation.


### SEQUENCE LISTING

<110> MEDIZINISCHE UNIVERSITÄT INNSBRUCK
   APEIRON BIOLOGICS FORSCHUNGS- UND ENTWICKLUNGSGESELLSCHAFT M.B.H.
<120> verfahren zur Erhöhung der Immunreaktivität
<130> r53419
<150> AT A 1996/2007
   <151> 2007-12-10
<160> 16
<170> Patentin version 3.4
<210> 1
   <211> 21
   <212> RNA
   <213> Artificial
<220>
   <223> RNAi
<400> 1
   gaacaucaca ggacuaugau u 21
<210> 2
   <211> 21
   <212> RNA
   <213> Artificial
<220>
   <223> RNAi
<400> 2
   ucauaguccu gugauguucu u 21
<210> 3
   <211> 21
   <212> RNA
   <213> Artificial
<220>
   <223> RNAi
<400> 3
   guacuggucc guuagcaaau u 21
<210> 4
   <211> 21
   <212> RNA
   <213> Artificial
<220>
   <223> RNAi
<400> 4
   uuugcuaacg gaccaguacu u 21
<210> 5
   <211> 21
   <212> RNA
   <213> Artificial
<220>
   <223> RNAi
<400> 5
   ggucgaauuu uggguauuau u 21
<210> 6
   <211> 21
   <212> RNA
   <213> Artificial
<220>
   <223> RNAi
<400> 6
   uaauacccaa aauucgaccu u 21
<210> 7
   <211> 21
   <212> RNA
   <213> Artificial
<220>
   <223> RNAi
<400> 7
   uaucagcauu uacgacuuau u 21
<210> 8
   <211> 21
   <212> RNA
   <213> Artificial
<220>
   <223> RNAi
<400> 8
   uaagucguaa augcugauau u 21
<210> 9
   <211> 21
   <212> RNA
   <213> Artificial
<220>
   <223> RNAi
<400> 9
   aaucaacucu gaacggaaau u 21
<210> 10
   <211> 21
   <212> RNA
   <213> Artificial
<220>
   <223> RNAi
<400> 10
   uuuccguuca gaguugauuu u 21
<210> 11
   <211> 21
   <212> RNA
   <213> Artificial
<220>
   <223> RNAi
<400> 11
   gacaaucccu cacaauaaau u 21
<210> 12
   <211> 21
   <212> RNA
   <213> Artificial
<220>
   <223> RNAi
<400> 12
   uuuauuguga gggauugucu u 21
<210> 13
   <211> 21
   <212> RNA
   <213> Artificial
<220>
   <223> RNAi
<400> 13
   uagcccaccu uauaucuuau u 21
<210> 14
   <211> 21
   <212> RNA
   <213> Artificial
<220>
   <223> RNAi
<400> 14
   uaagauauaa ggugggcuau u 21
<210> 15
   <211> 21
   <212> RNA
   <213> Artificial
<220>
   <223> RNAi
<400> 15
   ggagacacau uucggauuau u 21
<210> 16
   <211> 21
   <212> RNA
   <213> Artificial
<220>
   <223> RNAi
<400> 16
   uaauccgaaa ugugucuccu u 21